# EUROPÄISCHE PATENTSCHRIFT

(11) **EP 0 481 225 B1**
(45) Veröffentlichungstag und Bekanntmachung des Hinweises auf die Patenterteilung: **28.01.1998**
(21) Anmeldenummer: 91115707.1
(22) Anmeldetag: 17.09.1991
(51) Int. Cl.: B01J 20/26, A61L 15/00

(54) **Quellmittel und Wasserabsorptionsmittel auf Polymerbasis und ihre Verwendung zur Herstellung von Hygieneartikeln und zur Bodenverbesserung**
Swelling and water adsorbing agent based on polymer and its use in the manufacturing of sanitary articles and in soil amelioration
Agent de gonflement et d'adsorption d'eau à base du polymère et son utilisaton pour fabriquer des articles hygièniques et dans l'amélioration des sols

(30) Priorität: 19.09.1990 DE 4029592
(43) Veröffentlichungstag der Anmeldung: 22.04.1992
(73) Patentinhaber: Stockhausen GmbH & Co. KG, 47805 Krefeld (DE)
(72) Erfinder: Chmelir, Miroslav, Dr., W-4150 Krefeld (DE); Klimmek, Helmut, Dr., W-4150 Krefeld (DE)
(74) Vertreter: Klöpsch, Gerald, Dr.-Ing. Patentanwalt

(56) Entgegenhaltungen:
- EP-A- 0 071 063
- EP-A- 0 190 814
- DE-A- 2 650 377
- DE-A- 2 737 994
- DATABASE WPIL, Accession Nr. 81-55765D [31], Derwent Publications Ltd, London, GB;

## Beschreibung

Die Erfindung betrifft Polymerisate, die aus einer Kombination von synthetischen Polymeren und Naturpolymeren aus der Gruppe der Galaktomannane bestehen, die Wasser, wässrige Flüssigkeiten und Körperflüssigkeiten schnell aufnehmen, und ihre Verwendung zur Herstellung von Hygieneartikeln (z.B. Wegwerferzeugnissen wie Windeln, Damenbinden und Inkontinenzartikeln) und von wasserspeichernden Bodenverbesserungsmitteln.

Absorptionsmittel mit hohem Absorptionsvermögen für Wasser und Körperflüssigkeiten sind bekannt. Zu den vollsynthetischen Absorptionsmitteln gehören vernetzte Polymere und Copolymere auf Acryl- oder Methacrylsäurebasis (DE-OS 24 29 236, DE-OS 26 14 662, US-PS 4 018 951, US-PS 3 926 891, US-PS 4 066 583, US-PS 4 062 817, DE-OS 27 12 043, DE-OS 26 53 135, DE-OS 26 50 377, DE-OS 28 13 634), Maleinsäurederivate (nach US-PS 4 041 228) oder Acrylamidopropansulfonsäurecopolymerisate (nach DE-PS 31 24 008). Diese bekannten synthetischen Absorptionsmittel sind praktisch wasserunlöslich, absorbieren im Gleichgewicht das Vielfache ihres Gewichts an Wasser, Urin oder anderen wässrigen Lösungen, sind jedoch relativ beständig gegen biologische Abbaubarkeit.

Weitere Produkte wurden auf Stärkebasis, wie z.B. Stärke-Acrylnitril-Pfropfpolymerisate (nach US-PS 3 997 484, US-PS 3 661 815, US-PS 4 155 888, US-PS 3 935 099), gelatinisierte Stärkederivate (nach DE-OS 27 02 781) oder auf Cellulosebasis, wie Derivate von Alkyl- oder Hydroxyalkylcellulose (nach JP-PS 77/125 481), Carboxymethylcellulose (nach BE-PS 862 130, GB-PS 1 159 949) und auf Polysaccharidbasis (nach DE-OS 26 50 377) hergestellt. Diese Produkte, wie die mit Acrylnitril- oder Acrylsäure gepfropften Stärkepolymerisate gehören, zwar zu den abbaubaren Produkten, jedoch ist ihre Herstellung sehr aufwendig und die Menge des Naturprodukts im Endprodukt ist durch die hohe Viskosität des Reaktionsmediums, wie dies z.B. bei einer Monomerlösung mit gelöster Stärke der Fall ist, stark begrenzt.

Die natürlichen Quellmittel auf Polysaccharidbasis werden zwar in großen Mengen in der Lebensmittelindustrie oder Medizin verwendet, spielen aber als Absorptionsmaterial für Wasser und wässrige Lösungen nur eine untergeordnete Rolle, da sie nur ein geringes Absorptionsvermögen aufweisen. Gemäß GB 2 144 759 absorbiert eine Zusammensetzung aus Pektin (15 - 60 %) und 15 - 80 % cellulosehaltigem Material nur die zwei- bis fünffache Menge gegenüber üblichem Zellstoffmaterial. Vorteilhaft ist dagegen die biologische Abbaubarkeit solcher natürlichen Quellmittel, da die natürlichen Makromoleküle wie Zellulose, Stärke, Proteine usw. in biologischen Systemen durch Hydrolyse mit nachfolgender Oxidation in relativ kurzer Zeit abgebaut werden.

Das Aufnahmevermögen von Guargummi kann man gemäß US-PS 4 624 868 durch Vernetzung mit Borax erheblich verbessern, aber man muß in sehr verdünnten Lösungen (1 - 2 Gew.-% Guarmehl in Wasser) arbeiten, so daß durch die anschließend notwendige Verdampfung von großen Mengen an Wasser ein solches Verfahren unwirtschaftlich ist.

Mit Boratanion vernetztes Guargummi als Absorptionsmittel in einer Windelkonstruktion mit einem Aufnahmevermögen von 10 ml/g (Wasser) ist in der US-PS 3 903 889 und mit einem Aufnahmevermögen von 20 ml/g in der US-PS 3 070 095 beschrieben. Eine physikalische Mischung aus linearem oder verzweigtem, wasserlöslichem Polyacrylsäuresalz (Molmasse 10.000 bis 10.10⁶) oder teilweise hydrolysiertem Pfropf-Mischpolymerisat aus Stärke und Acrylamid mit Guarangummi, Xanthangummi oder Alginate bringt gemäß DE-OS 26 50 377 auch ein verbessertes Aufnahmevermögen von synthetischem Urin bzw. Pferdeblut als Prüfflüssigkeit. In diesen Fällen sind aber beide Komponenten wasserlöslich und dadurch z.B. für eine moderne Windelkonstruktion wegen der extrem hohen wasserextrahierbaren Anteile ungeeignet.

Eine weitere Verwendung von Guarmehl als Verdicker der Monomerlösung bei der durch ionisierende Strahlung initiierten Polymerisation wurde in der DE-OS 27 37 994 beschrieben. In diesem Fall werden Stärke, Xanthan, Guar oder auch andere Verdickungsmittel der Monomerlösung vor der Polymerisation zugesetzt, um eine gleichmäßige dünne Schicht der Monomerlösung in einem U-förmigen Bestrahlungsbehälter, vom oberen Behälterrand bis zum Boden, zu verfestigen und damit das Herunterfließen der verdickten Monomerlösung von den senkrechten Wänden des Behälters zu verhindern. Eine gleichmäßige Verteilung der Monomerlösung in dünner Schicht und in einem gleichmäßigen Abstand rund um die Strahlungsquelle herum ist unbedingt notwendig, um eine gute Effektivität der energiereichen Strahlung zu erreichen. Zur Verwendung als Absorptionsmittel für wässrige Flüssigkeiten werden die mit mehrfunktionellen Verbindungen vernetzten Produkte als weniger geeignet bezeichnet, verglichen mit solchen, die durch energiereiche Strahlung vernetzt (C-C-Vernetzung) werden.

Guarmehl in Kombination mit Carrageenan, Agar oder Xanthan als Verdicker für Nahrungsmittel wird in der DE-OS 33 35 593 und als Verdicker bei der Düngemittelherstellung in der GB-PS 1 437 266 beschrieben.

Die medizinische Anwendung von Guarmehl beruht darauf, daß das Guarmehl nach oraler Einnahme nicht enzymatisch hydrolysiert und daher nicht resorbiert wird, es verzögert die Resorption von Nährstoffen, wirkt appetithemmend, senkt den Blutcholesteringehalt und reguliert die Darmperistaltik. Da aber der Quellvorgang beim handelsüblichen Guarmehl enorm schnell ist, wobei eine schleimige, hochviskose und klebrige Masse entsteht, besteht bei einer oralen Einnahme im trockenen Zustand eine erhebliche Erstickungsgefahr, denn das gequollene Guarmehl kann Mund, Rachen und Speiseröhre verkleben. Die Einnahme im flüssigen Zustand erfordert wieder wegen der hohen Viskosität der Lösungen große Mengen an Flüssigkeit (400 ml auf 4 g Guarmehl), die zusätzlich eingenommen werden müssen. Gemäß EP-OS 0 241 710 wird eine langsamere Quellung durch Verwendung von grobgemahlenem Guarmehl (Korngröße 50 - 1500 µm) erreicht.

Die EP 0 071 063 betrifft ein Absorptionsmittel für Blut und seröse Körperflüssigkeiten sowie ein Verfahren zu seiner Herstellung, das eine Kombination von wasserquellbaren, gegebenenfalls mit mehrfunktionellen Verbindungen vernetzten, synthetischen Polymerisaten als Komponente A mit einer wasserlöslichen Verbindung als Komponente B darstellt. Als geeignete Komponente B werden keine Polysaccharide, sondern nur Mono- oder Oligosaccharide erwähnt.

Aufgabe der Erfindung ist die Schaffung von Absorptionsmitteln, die die Vorteile des hohen Absorptionsvermögens für Wasser und Körperflüssigkeiten der synthetischen Absorptionsmittel ohne deren Beständigkeit gegen biologischen Abbau aufweisen.

Diese Aufgabe wird erfindungsgemäß durch eine Mischung gelöst, die aus mindestens zwei Komponenten A und B besteht, wobei die Komponente A wenigstens ein wasserquellbares, synthetisches, mit einer mehrfunktionellen Verbindung vernetztes Polymeres oder Copolymeres und die Komponente B wenigstens ein Polysaccharid aus der Gruppe der Galaktomannane bzw. Polygalaktomannane sowie Galaktomannanderivate oder deren Abmischungen mit anderen natürlichen oder synthetischen Polymerisaten, die bei normaler Temperatur als rieselfähige Pulver oder als Fasermaterialien vorliegen und in Wasser nur begrenzt oder unlöslich sind, ist.

Überraschenderweise hat sich gezeigt, daß der Zusatz eines Polysaccharids aus der Gruppe der Galaktomannane wie z. B. Guarmehl, das hauptsächlich aus Galaktomannan besteht und selbst nur ein mäßiges Quellvermögen für synthetischen Modellurin hat (5 - 6 ml/g) zu einem anderen synthetischen vernetzten Polymerisat (Komponente A) das Aufnahmevermögen der Mischung aus den Komponenten A und B erheblich erhöht.

Das Mischungsverhältnis der beiden Komponenten A und B kann in weiten Bereichen schwanken. So kann das erfindungsgemäße Absorptions- und Quellmittel die Komponente A zu 20 - 98 Gew.-%, vorzugsweise 50 - 90 Gew.-%, und die Komponente B in Mengen von 2 - 80 Gew.-%, vorzugsweise 10 - 50 Gew.-%, enthalten.

Die Herstellung des aus Komponenten A und B zusammengesetzten Endprodukts kann im einfachsten Fall durch eine einfache Abmischung der beiden Komponenten in getrocknetem (pulverigem) oder angefeuchtetem bzw. schwach gequollenem Zustand der einen oder beider Komponenten erfolgen.

Gemäß einer weiteren Ausführungsform der Erfindung kann man die Komponente B auch in Pulverform oder in schwach gequollener Form dem synthetischen Polymerisat A während seines Herstellungsprozesses zusetzen. Dann entsteht als Endprodukt ein Absorptions- und Quellmittel, in dem die Komponente B in das synthetische Polymerisat der Komponente A durch eine chemische Reaktion und/oder physikalische Wechselwirkung eingebunden wird. Hierdurch werden die mit Wasser extrahierbaren Anteile im Endprodukt gegenüber einer physikalischen Abmischung der beiden Komponenten deutlich erniedrigt. Bei dieser Ausführungsform wird die Komponente B in getrocknetem Zustand als Pulver oder in leicht angefeuchtetem bis gequollenem Zustand bevorzugt erst in der Endphase der Herstellung der Komponente A, d.h. erst nachdem ein Polymerisationsumsatz von mehr als 60 %, bevorzugt mehr als 90 %, erreicht wird, zu dem gequollenen Polymergel der Komponente A zugesetzt und danach weiterverarbeitet. In der Regel wird die Komponente B bis zu einem Polymerisationsumsatz von 95 % zugesetzt werden, sie kann jedoch auch bei höheren Polymerisationsumsätzen bis zu 99 % zugegeben werden.

Die Herstellung der Komponente A als gequollenes Polymergel kann diskontinuierlich in einem Polymerisationsgefäß oder kontinuierlich auf einem endlosen Band erfolgen. Zum Beispiel wird gemäß DE-PS 35 44 770 die Polymerisation in einer wässrigen Lösung, enthaltend das wasserlösliche Monomere und evtl. die Comonomeren in einer Konzentration von 2,2 bis 8,3 Mole polymerisationsfähiger Doppelbindungen pro Kilo Monomerenlösung, insbesondere 3,5 bis 6,25 Mole (entsprechend 16 - 60, insbesondere 25 - 45 Gew.-% Acrylsäure, wenn diese als Monomeres verwendet wird), und im Temperaturbereich von etwa 10 - 120°C durchgeführt.

Als Komponente A kommen in Frage in erster Linie die Polymerisate von Acrylsäure und Methacrylsäure alleine als Homopolymerisat oder als Copolymerisat, ferner aber auch die Polymerisate von anderen wasserlöslichen Monomeren, wie (Meth-)Acrylamid und (Meth-)Acrylnitril, Vinylpyridin, Vinylpyrrolidon, Vinylacetat sowie weitere wasserlösliche Monomere, wie polymerisationsfähige Säuren und ihre Salze, insbesondere die Malein-, Fumar-, Itacon-, Vinylsulfon- oder 2-Acrylamido-2-methylpropansulfonsäure; ferner hydroxygruppenhaltige Ester polymerisationsfähiger Säuren, insbesondere die Hydroxyethyl- und Hydroxypropylester der Acryl- und der Methacrylsäure verwendet werden können; weiter aminogruppenhaltige und ammoniumgruppenhaltige Ester und Amide polymerisationsfähiger Säuren wie die Dialkylaminoester, insbesondere die Dimethyl- und die Diethylaminoalkylester der Acryl- und der Methacrylsäure sowie die Trimethyl- und die Triethylammoniumalkylester sowie die entsprechenden Amide.

Die vernetzenden Monomeren, wie z.B. Monomere mit mehr als einer polymerisationsfähigen Gruppe im Molekül, werden in geringen Anteilen zusammen mit vorstehenden Monomeren polymerisiert, wobei vernetzte Homo- oder vernetzte Copolymerisate entstehen.

Wenn die vorstehenden Monomeren alleine nur mit Vernetzerzusatz (in kleinen Mengen,verglichen mit der Monomerkonzentration) polymerisiert werden, entstehen vernetzte Homopolymerisate. Bei der Polymerisation eines Gemisches von verschiedenen Monomeren und einer kleinen Menge eines Vernetzers werden die entstandenen Produkte als vernetzte Copolymerisate bezeichnet.

In geringen Mengen können noch wasserunlösliche Monomere copolymerisiert werden wie die Ester der Acryl- und/oder Methacrylsäure mit C₁-C₁₀-Alkoholen, Styrol und alkylierte Styrole. Im allgemeinen liegt der Anteil an den wasserlöslichen Monomeren bei 40 bis 100 Gew.-%, bezogen auf die Gesamtheit der Monomeren. Der Anteil an den vernetzenden Comonomeren liegt bei 0,01 bis 20 Gew.-%, vorzugsweise 0,01 bis 2,0 Gew.-%, bezogen auf die Gesamtheit der Monomeren. Die wasserunlöslichen (hydrophoben) Monomeren haben in der Regel einen Anteil von 0 bis 40 Gew.-% der Monomeren.

Als vernetzende Monomere seien bi- oder mehrfunktionelle Monomere, z.B. Amide wie das Methylenbisacryl- bzw. -methacrylamid oder Ethylenbisacrylamid, ferner Ester ungesättigter, ein- und mehrwertiger Carbonsäuren von Polyolen, wie Diacrylate oder Triacrylate, z.B. Butandiol- oder Ethylenglykoldiacrylat, bzw. -methacrylat, Trimethylolpropantriacrylat, ferner Vinylmethacrylat und Allylverbindungen wie Allyl(meth)acrylat, Triallylcyanurat, Maleinsäurediallylester, Polyallylester, Tetraallyloxiethan, Triallylamin, Tetraallylethylendiamin, Allylester der Phosphorsäure bzw. phosphorigen Säure, ferner vernetzungsfähige Monomere, wie die N-Methylolverbindungen von Amiden wie dem Methacrylamid bzw. Acrylamid und die davon abgeleiteten Äther.

Die Polymerisation kann durch chemische Katalyse und/oder durch energiereiche Strahlung/Licht initiiert werden. Als geeignete Katalysatoren können z.B. Peroxiverbindungen wie Kaliumperoxidisulfat, Wasserstoffperoxid, organische Peroxide wie Benzoylperoxid, tert. Butylhydroperoxid, tert. Butylperpivalat, Redox-Systeme, wie z.B. Kaliumperoxidisulfat-Natriumdisulfit, Wasserstoffperoxid-Hydroxylaminchlorid oder Azoinitiatoren wie AIBN (2,2'-Azobis-(isobutyronitril)) oder 2,2'-Azobis(2-amidinopropan)dihydrochlorid verwendet werden. Als Photoinitiatoren können beispielsweise Benzoin und seine Derivate, z.B. Benzoinether, wie Benzoin-Ethyl-Propyl-Ether und Benzil und seine Derivate, wie Benzilketale oder Acryldiazoniumslaze, Acetophenonderivate und andere alleine oder in Gemischen und/oder auch in Gemischen mit peroxidhaltigen Katalysatorsystemen oder Azoinitiatoren verwendet werden. Im allgemeinen liegt der Gehalt an Photoinitiatoren bei 0,002 bis 2,0 Gew.-%, vorzugsweise 0,01 bis 0,2 Gew.-%, bezogen auf die eingesetzten Monomeren. Der Gehalt an Katalysatoren liegt im allgemeinen bei 0,02 bis 5,0 Gew.-%, vorzugsweise zwischen 0,20 bis 2,0 Gew.-%, bezogen auf die Monomeren.

Als Komponente B werden die Polysaccharide aus der Gruppe der Galaktomannane, die z.B. im Guarmehl oder Johannisbrotkernmehl enthalten sind, alleine oder in Abmischungen mit anderen natürlichen Polymerisaten auf Polysaccharidbasis, wie Cellulose und ihre Derivate (z.B. Alkyl-, Hydroxyalkyl-, Carboxymethylcellulose), Viskosefaser, Gummiharze (z.B. Tragacanthgummi, Gummi Arabicum, Pektin Dextran u.a.), Stärke und Stärkederivate, wie z.B. Mais-, Korn-, Kartoffelstärke, Amylose, Amylopektin, Dextrin, modifizierte Stärke, Hydroxyethylstärke, kationische Stärke, Stärkepfropfpolymerisate u.a.) verwendet.

Neben diesen Naturpolymeren können zur Abmischung mit der Komponente A auch andere Materialien mit großer Oberfläche wie z.B. Fasermaterial aus Naturfaser, bevorzugt Baumwolle-, Wolle- und Seidenfasern, weiterhin Fasermaterial aus Cellulosefasern wie Viskose-, Acetat-und Triacetatfasern oder aus synthetischen Fasern auf der Basis von Polyester, Polyolefinen, Polyacrylnitril, Polyamid, Polyvinylalkohol, -acetat und -chlorid, Polyurethan, Polyvinylharnstoff und der Copolymerisate von diesen Polymeren, verwendet werden. Die Fasermaterialien können bevorzugt als Kurzschnittfaser zusammen mit den genannten Galaktomannanen in die Komponente A eingearbeitet werden.

Durch weitere Verarbeitung der Abmischung der beiden Komponenten in einem Mischer mit rotierendem Mischwerkzeug und nachträglicher Trocknung der Polymergelmasse erhält man ein Endprodukt, in dem das Guarpolymer (Komponente B) im synthetischen Polymerisat gebunden ist, so daß die mit Wasser extrahierbaren Anteile des Endproduktes deutlich niedriger sind als bei einem physikalischen Gemisch der beiden Komponenten. Je nach dem verwendeten Gehalt an der zum Teil in Wasser löslichen Komponente B bekommt man Endprodukte, die lösliche Anteile unter 30 Gew.-%, bevorzugt unter 20 Gew.-%, aufweisen. Bevorzugt wird die Trocknung im Bereich von 50 - 160°C vorgenommen.

Die Einbindung der Komponente B in das synthetische Polymerisat kann durch Zusatz von verschiedenen, oben schon erwähnten Katalysatoren in einer Menge von 0,01 bis 2,0 Gew.-% und/oder oben bereits erwähnten mehrfunktionellen Monomeren in einer Menge von 0,05 bis 5,0 Gew.-% zu der Komponente B oder Komponente A verstärkt werden. Die Katalysatoren oder mehrfunktionellen Verbindungen können z.B. als Lösung auf die Komponenten A oder B vor oder während der Abmischung aufgesprüht werden.

Die Abmischung der beiden Komponenten kann in einer geeigneten Mischeinrichtung erfolgen. Der in dieser Beschreibung benutzte Mischer bestand aus einem vertikal oder horizontal gelegenen Metallzylinder, dessen Mischwerkzeug mit Leitschaufeln ausgerüstet war, welche die Wände des Mischerzylinders gründlich abstreifen. Nachdem eine bestimmte Drehzahl des Rührwerks erreicht wurde, erfolgte auf der ganzen Länge des Metallzylinders eine gleichmäßige Durchmischung und Durchknetung der beiden Komponenten.

Das Endprodukt besteht aus den Komponenten A und B in einem Gewichtsverhältnis 20 bis 98 Gew.-%, vorzugsweise 50 bis 90 Gew.-%, der Komponente A, zu 2 bis 80 Gew.-%, vorzugsweise 10 bis 50 Gew.-%, der Komponente B.

Die nach dem erfindungsgemäßen Verfahren aus Komponenten A und B hergestellten Endprodukte zeigen ein deutlich verbessertes Aufnahmevermögen für synthetische Modellurinlösung, wobei auch die Abbaubarkeit der Endprodukte verbessert wurde. Durch die Abmischung der beiden Komponenten A und B wird eine synergestische Wirkung erzielt, die sich vor allem im erhöhten Retentionswert, d.h. in erhöhter Absorption unter der Belastung auswirkt. Die Absorption unter Druck und Belastung gehört zu den wichtigsten Kriterien bei der Beurteilung eines Superabsorbers.

### Prüfmethoden

1) Zur Ermittlung der Aufnahmegeschwindigkeit wird die Aufnahme von Modellurin nach dem Demand-Absorbency-Test (DAT-Methode nach W. F. Schlauch, Vortrag Index 1978, Amsterdam) durchgeführt und die Aufnahmegeschwindigkeit nach 60 Sekunden sowie die Maximalaufnahme und Retention ermittelt. Das Meßgerät besteht aus einer Bürette, die mit der Modellurinlösung (2,0 % Harnstoff, 0,9 % NaCl, 0,1 % MgSO₄ und 0,06 % CaCl₂, aufgelöst in destilliertem Wasser) gefüllt ist, und einem Probetisch, der mit einer an die Meßbürette angeschlossenen Öffnung für den Modellurinlösungsaustritt versehen ist. Auf dem mit einem dünnen Vlies (10 x 13,5 cm) bedeckten Probetisch werden 0,5 g des erfindungsgemäßen Produktes, vermischt mit 5 mg Aerosil 200, in Form einer kreisrunden Fläche von 4,5 cm Durchmesser, zentrisch über dem Flüssigkeitsaustritt, gleichmäßig aufgestreut. Durch Schließen des Schlauches und leichte Druckgebung wird der Kontakt der Modellurinlösung mit dem Pulverprodukt hergestellt, so daß die Modellurinlösung durch das erfindungsgemäße Produkt absorbiert werden kann. Die absorbierte Menge der Modellurinlösung wird nach 20 - 30 Minuten als Maximalwert abgelesen. Anschließend wird die Retention durch Belastung des gequollenen Geles mit einem Gewicht von 10 g/cm² ermittelt; die Zeit der Belastung beträgt 5 Minuten. Die ermittelten Retentionswerte sind in den Beispielen tabellenweise zusammengefaßt.
2) Als weitere Methode zur Bestimmung der Flüssigkeitsaufnahmegeschwindigkeit wurde ein Teebeuteltest durchgeführt, wobei die Flüssigkeitsaufnahme von 0,2 g Prüfsubstanz ohne Aerosilzusatz in einem Teebeutel nach 10 Minuten (Maximalwert) und nach dem Schleudern in einer Zentrifuge, beispielsweise in einer handelsüblichen Wäscheschleuder bei 1400 Upm gravimetrisch ermittelt und auf 1 g Produkt umgerechnet wurde (Retentionswert). Als Prüfflüssigkeit wurde die wässrige 0,9%ige NaCl-Lösung verwendet.
3) Künstliche Abbaubedingungen durch dem Tageslicht ähnliche Bestrahlung wurden mit Xenotestlampe und Bestrahlungszeiten von 30, 60 und 90 Min. an mit Wasser gequollenem Polymergel (200 g Wasser auf 1 g Produkt) simuliert. Das gequollene Polymergel wurde mit der Xenotestlampe bestrahlt und nach bestimmten Zeiten wurde die Abbaubarkeit des Polymergels nach einer Skala mit Stufen 1 bis 8 beurteilt (s. Beispiel 21).
   - Stufe 1:: Gelstruktur unverändert
   - Stufe 2:: Gelstruktur leicht verändert
   - Stufe 3:: Gelstruktur noch erkennbar, aber leichtes Fließen
   - Stufe 4:: Gelstruktur stark verflossen
   - Stufe 5:: Gelstruktur nicht mehr erkennbar, hochviskose Flüssigkeit
   - Stufe 6:: leichtviskose Flüssigkeit
   - Stufe 7:: wasserähnliche Flüssigkeit
   - Stufe 8:: Wasser verdampfte vollständig

   Die Xenotestlampe entspricht im spektralen Bereich annähernd dem Tageslicht,und die Methode wurde von den Cassella Farbwerken, Mainkur AG, Frankfurt, entwickelt.

Die Erfindung wird durch die nachfolgenden Beispiele erläutert:

### Beispiele 1 bis 9:

Ein mit 0,7 Gew.-% Methylenbisacrylamid vernetztes Acrylsäurepolymerisat, das zu ca. 70 % als Natriumsalz in einer Kornfraktion 100 bis 650 µm vorlag (Komponente A), wurde mit der Komponente B, feingemahlenem Guaran Polymer KWL 2000 (Roeper, Hamburg) in verschiedenen Verhältnissen vermischt und das Aufnahmevermögen der Mischung nach der DAT-Methode mit synthetischem Modellurin ermittelt. Die Ergebnisse sind in der Tabelle 1 zusammengefaßt, wobei auch die Werte für die einzelnen Komponenten als Vergleichsbeispiele in der Tabelle 1 aufgeführt sind.

Durch die Abmischung der beiden Komponenten erzielt man eine synergistische Wirkung, die sich vor allem im erhöhten Retentionswert, d.h. in erhöhter Absorption unter der Belastung auswirkt. Die Absorption unter Druck und Belastung gehört zu den wichtigsten Kriterien bei der Beurteilung eines Superabsorbers.

Wie aus der Tabelle 1 zu entnehmen ist, ändert sich der Retentionswert der Abmischung bei steigendem Zusatz des Guar-Polymers, das selbst nur einen recht niedrigen Absorptionswert (4,8 ml/g) hat, zur Komponente A in einem Konzentrationsbereich bis zu 33 Gew.-% nur wenig und liegt sogar etwas höher als bei der Komponente A allein. Umgerechnet auf den Gehalt der Komponente A in der Mischung ohne oder mit Berücksichtigung der Absorptionswerte des Guarpolymers (Ret^{(b)} und Ret^{(c)} zeigt sich eine deutliche Verbesserung der Retentionswerte in der Abmischung.

### Bemerkungen (zu den Tabellen 1 und 2):

Die DAT-Werte Max^{(a)} und Ret^{(a)}
   beziehen sich auf 1 g des zusammengesetzten Produktes aus den beiden Komponenten A und B;
die DAT-Werte Max^{(b)} und Ret^{(b)}
   beziehen sich auf 1 g der Komponente A im Produkt, wobei der Einfluß von Komponente B auf die Absorptionswerte vernachlässigt wurde;
die DAT-Werte Max^{(c)} und Ret^{(c)}
   beziehen sich auf 1 g der Komponente A im Produkt, wobei aber der Einfluß der Komponente B auf die Absorptionswerte rechnerisch berücksichtigt wurde.

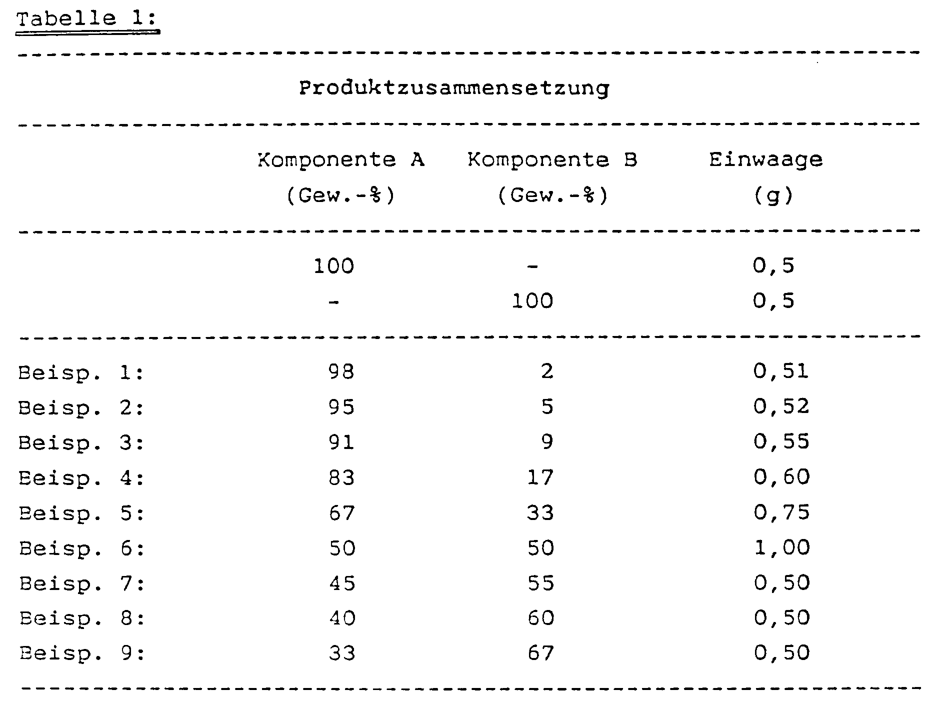

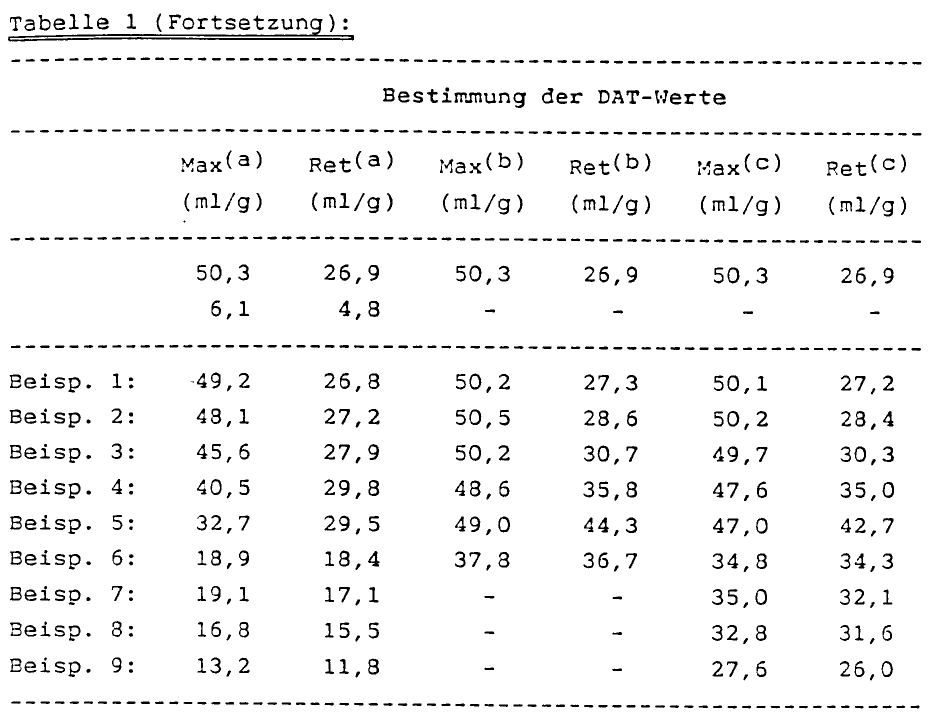

### Beispiele 10 bis 13:

Das vernetzte Acrylsäurepolymerisat aus den Beispielen 1 bis 9 wurde in einer Menge von 50 g in der gleichen Gewichtsmenge an Wasser/Methanol-Gemisch (25:75) suspendiert und dadurch vorgequollen. Zu der Suspension wurde dann die Komponente B (Guaran-Polymer KWL 2000) zugesetzt und 15 Min. gerührt. Danach wurde die Suspension abgenutscht und bei 50°C im Vakuum getrocknet. Die ermittelten Ergebnisse sind in Tabelle 2 zusammengefaßt.

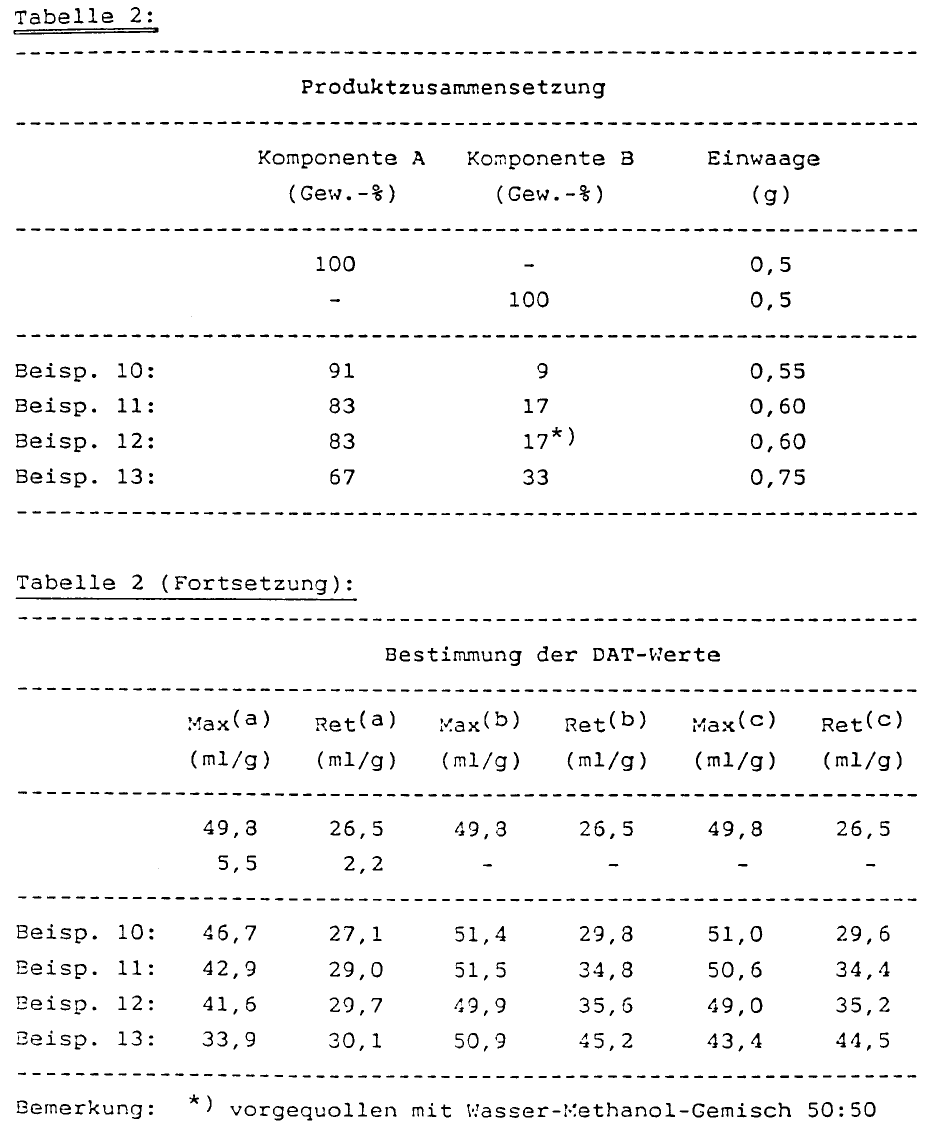

### Beispiel 14:

Die Komponente A (1 g) gemäß Beispielen 1 bis 13 wurde mit 0,25 g einer Abmischung der Komponente B (Guaran-Polymer/Carboxymethylcellulose 50 : 50) vermischt und das Absorptionsvermögen nach der DAT-Methode ermittelt. Das Endprodukt zeigte einen Maximalwert von 58,2 ml und einen Retentionswert von 45,8 ml. Zum Vergleich: Die Komponente A alleine weist einen Maximalwert von 50,2 ml/g und eine Retention von 26,5 ml/g auf.

### Beispiele 15 bis 17:

In einem Polymerisationsgefäß wurden 410 g Acrylsäure, 2,6 g Triallylamin (0,6 Gew.-%, bezogen auf Acrylsäure) in 670 ml Wasser gelöst und mit 376 g Natronlauge (45%ig) teilneutralisiert. Bei Raumtemperatur wurden die Katalysatorkomponenten 0,15 g Benzildimethylketal (Irgacure 651, Ciba-Geigy) und 1,5 g Natriumperoxidisulfat, gelöst in 25 ml Wasser, zugegeben und die adiabatische Polymerisation gestartet. Das entstandene Polymergel wurde zerkleinert, mit 2000 ppm (bezogen auf Trockensubstanz des Polymergels) Natriumperoxidisulfat, gelöst in 30 ml Wasser, gleichmäßig besprüht und danach in einem Mischer, dessen rotierendes Mischwerkzeug mit Leitschaufeln, die die Wände des Mischerzylinders gründlich abstreifen, ausgerüstet ist, mit verschiedenen Mengen an Guaran-Polymer 30 Minuten lang verarbeitet. Die entstandene Polymergelmasse wurde dann bei 120°C getrocknet. Das Aufnahmevermögen des Endproduktes wurde nach dem Teebeutel-Test ermittelt (s. Tabelle 3).

**Tabelle 3**

| Produktzusammensetzung | | | | | |
|---|---|---|---|---|---|
| | Komponente A | Komponente B | Teebeuteltest-Werte | | |
| | Acrylsäurepolymerisat Gew.-% | Guaran Polymer Gew.-% | Maximal (ml/g) | Retention | |
| | | | | (a) (ml/g) | (b) (ml/g |
| | 100 | 0 | 41,0 | 27,1 | 27,1 |
| | 0 | 100 | 6,0 | 2,5 | -- |
| Beisp. 15: | 80 | 20 | 37,9 | 27,3 | 34,1 |
| Beisp. 16: | 60 | 40 | 38,2 | 23,5 | 39,2 |
| Beisp. 17: | 50 | 50 | 38,1 | 22,7 | 45,4 |
| Bemerkung: Die Teebeuteltest-Werte Maximal und Retention (a) beziehen sich auf 1 g des zusammengesetzten Produktes aus Komponenten A und B, die Retention (b) bezieht sich auf 1 g der Komponente A im Produkt. | | | | | |

### Beispiele 18 bis 20:

In einem Polymerisationsgefäß wurden 237 g Acrylsäure, 357 g Acrylamid und 2,3 g N,N'-Methylenbisacrylamid in 975 ml Wasser gelöst und mit 371 g Kalilauge (45%ig) neutralisiert. Bei Raumtemperatur wurden die Katalysatorkomponenten (0,26 g Azobisamidinpropandihydrochlorid, 0,06 g Irgacure 651 und 0,26 g t-Butylhydroperoxid, gelöst in Wasser, zugegeben und die adiabatische Polymerisation durch UV-Licht gestartet. Der erreichte Umsatz betrug 99,5%. Das entstandene Copolymergel wurde zerkleinert, in einer Mischeinrichtung wie in den Beispielen 15 bis 17 mit Guaran-Polymer und Polyamidkurzschnittfasern (1,0 mm, 6,7 dtex) in verschiedenen Verhältnissen vermischt und danach getrocknet. Zusätzlich wurde noch die Aufnahmegeschwindigkeit als Teebeuteltest-Wert nach 15 Sekunden Tauchzeit ermittelt (Ergebnisse siehe Tabelle 4). Die Einarbeitung der Polyamidfasern zusammen mit dem Guaran-Polymer in das vernetzte Copolymerisat bewirkte eine schnellere Aufnahme der Prüfflüssigkeit.

**Tabelle 4**

| | Komponente A | Komponente B | | Teebeuteltest-Werte | | | | |
|---|---|---|---|---|---|---|---|---|
| | Acrylsäure/acrylamidcopolymerisat Gew.-% | Guaran-Polymer Gew.-% | Polyamidfasern Gew.-% | Maximal | | Retention | | 15 Sek. -Wert (ml/g) |
| | | | | (a) | (b) | (a) | (b) | |
| | | | | (ml/g) | | (ml/g) | | |
| | 100 | 0 | 0 | 39,5 | 39,5 | 27,3 | 27,3 | 11,0 |
| Beisp. 18: | 80 | 20 | 0 | 35,8 | 44,7 | 24,3 | 30,4 | 10,9 |
| Beisp. 19: | 80 | 10 | 10 | 36,6 | 45,7 | 22,0 | 27,5 | 14,2 |
| Beisp. 20: | 70 | 20 | 10 | 34,3 | 49,0 | 19,6 | 28,0 | 16,4 |
| Bemerkung: Die Teebeuteltest-Werte Maximal (a) und Retention (a) beziehen sich auf 1 g des zusammengesetzten Produktes aus den Komponenten A und B, Maximal (b) und Retention (b) beziehen sich auf 1 g der Komponente A im Produkt. | | | | | | | | |

### Beispiel 21:

Das pulverförmige Produkt aus den Beispielen 15 bis 17 wurde in Wasser gequollen (200 g Wasser auf 1 g Produkt) und anschließend als gequollenes Gel mit der Xenotextlampe bestrahlt, um Tageslichtabbaubedingungen zu simulieren. Nach 30, 60 und 90 Minuten wurde die Abbaubarkeit des Polymergels nach einer Skala mit Stufen 1 bis 8 beurteilt. Die Ergebnisse sind in der Tabelle 5 zusammengefaßt, aus der die verbesserte Abbaubarkeit der zusammengesetzten Produkte ersichtlich ist.

**Tabelle 5**

| | Komponente A | Komponente B | Belichtungszeit | | |
|---|---|---|---|---|---|
| | Acrylsäurepolymerisat Gew.-% | Guaran Polymer Gew.-% | Minuten | | |
| | | | 30 | 60 | 90 |
| | 100 | 0 | 2 | 4 | 5 |
| Beisp. 15: | 80 | 20 | 5 | 6 | 6 |
| Beisp. 16: | 60 | 40 | 5 | 6 | 7 |
| Beisp. 17: | 50 | 50 | 6 | 6 | 7 |

- Stufe 1:: Gelstruktur unverändert
- Stufe 2:: Gelstruktur leicht verändert
- Stufe 3:: Gelstruktur noch erkennbar, aber leichtes Fließen
- Stufe 4:: Gelstruktur stark verflossen
- Stufe 5:: Celstruktur nicht mehr erkennbar,
hochviskose Flüssigkeit
- Stufe 6:: leichtviskose Flüssigkeit
- Stufe 7:: wasserähnliche Flüssigkeit
- Stufe 8:: vollständige Verdampfung von Wasser

## Patentansprüche

1. Absorptionsmittel und Quellmittel für Wasser, wäßrige Lösungen und Körperflüssigkeiten, bestehend aus einer physikalischen Mischung aus mindestens zwei Komponenten, wobei die Komponente A wenigstens ein wasserquellbares, synthetisches, mit einer mehrfünktionellen Verbindung vernetztes Polymeres oder Copolymeres ist, dadurch gekennzeichnet, daß die Komponente B wenigstens ein Polysaccharid aus der Gruppe der Galaktomanane bzw. Polygalaktomanane sowie Galaktomananderivate oder deren Abmischungen mit anderen natürlichen oder synthetischen Polymerisaten, die bei normaler Temperatur als rieselfähige Pulver oder als Fasermaterialien vorliegen und in Wasser nur begrenzt löslich oder unlösliche sind, ist.

2. Absorptionsmittel und Quellmittel für Wasser, wässrige Lösungen und Körperflüssigkeiten, erhältlich aus mindestens zwei Komponenten A und B, wobei die Komponente A wenigstens ein wasserquellbares, synthetisches, mit einer mehrfunktionellen Verbindung vernetztes Polymeres oder Copolymeres und die Komponente B wenigstens ein Polysaccharid aus der Gruppe der Galaktomannane bzw. Polygalaktomannane sowie Galaktomannanderivate oder deren Abmischungen mit anderen natürlichen oder synthetischen Polymerisaten, die bei normaler Temperatur als rieselfähige Pulver oder als Fasermaterialien vorliegen und in Wasser nur begrenzt oder unlöslich sind, ist, durch Einbringen der Komponente B in getrocknetem Zustand oder in leicht angefeuchtetem bis gequollenem Zustand während der Herstellung der Komponente A, nachdem deren Polymerisationsumsatz mehr als 60 %, bevorzugt mehr als 90 %, erreicht hat, sowie nachfolgender Trocknung.

3. Absorptionsmittel und Quellmittel nach Anspruch 1 oder 2, dadurch gekennzeichnet, daß die Komponente A ein vernetztes Polymeres oder Copolymeres auf Basis von Acrylsäure, Methacrylsäure, Derivaten dieser Carbonsäuren, vorzugsweise ein vernetztes Homo- oder Copolymeres der Acryl-, Methacryl-, Acrylamidopropansulfonsäure, den Alkali- oder Ammoniumsalzen dieser Carbonsäuren, des Acryl- oder Methacrylamids und deren Derivaten, des Vinylpyrrolidons sowie deren Copolymeren untereinander oder mit anderen nur teilweise wasserlöslichen Monomeren wie z.B. Vinylacetat ist.

4. Absorptionsmittel und Quellmittel nach Anspruch 1 oder 2, dadurch gekennzeichnet, daß die Komponente B ein Guarmehl oder Johannisbrotkernmehl alleine oder in Abmischung mit anderen Polysacchariden oder Polysaccharidderivaten, bevorzugt Abmischungen von Guar mit Stärke, Dextrin, Dextran, Cellulose und deren Derivaten ist.

5. Absorptionsmittel und Quellmittel nach Anspruch 1 oder 2, dadurch gekennzeichnet, daß die Komponente B ein Guarmehl oder Johannisbrotkernmehl in Abmischung mit Natur- oder synthetischen Fasern, bevorzugt Abmischungen mit Kurzschnittfasern auf Cellulosebasis, insbesondere Abmischungen mit Baumwoll- oder Viskosefasern, oder auf Polymerbasis, insbesondere Abmischungen mit Polyester-, Polyacrylnitril- oder Polyamidfasern, ist.

6. Absorptionsmittel und Quellmittel nach Anspruch 2, dadurch gekennzeichnet, daß die Komponente B in dem synthetischen Polymeren (Komponente A) eingebunden ist, so daß die mit Wasser extrahierbaren Anteile des Endproduktes niedriger als bei einer Abmischung, bevorzugt niedriger als 30 Gew.-%, bevorzugt niedriger als 20 Gew.-%, sind, bezogen auf Komponente B.

7. Absorptionsmittel und Quellmittel nach einem der Ansprüche 1 oder 2, dadurch gekennzeichnet, daß das Endprodukt 20 bis 98 Gew.-%, vorzugsweise 50 bis 90 Gew.-%, an Komponente A und 2 bis 80 Gew.-%, vorzugsweise 10 bis 50 Gew.-%, an Komponente B enthält.

8. Verwendung des Absorptionsmittels nach Anspruch 1 bis 7 zur Aufnahme und/oder Zurückhaltung von Wasser und/oder wässrigen Lösungen, insbesondere von wässrigen und serösen Körperflüssigkeiten wie Urin oder Blut, in absorbierenden Wegwerferzeugnissen für hygienische, chirurgische und medizinische Zwecke wie Babywindeln, Tampons und Damenbinden.

9. Verwendung des Absorptionsmittels nach Anspruch 1 bis 7 für medizinische Zwecke wie zur oralen Verabfolgung als Quellmittel, als Zusatz zu Kontrastmitteln, Regulierung der Darmperistaltik sowie zur oralen Verabfolgung zwecks Verzögerung der Resorption bzw. Wirkung von eingenommenen Medikamenten oder zwecks Senkung des Blutcholesterinspiegels.

10. Verwendung des Absorptionsmittels nach Anspruch 1 bis 7 zur Aufnahme und/oder Zurückhaltung von Wasser oder wässrigen Lösungen und zur nachfolgenden, gesteuerten, verzögerten Abgabe der absorbierten Flüssigkeit sowie ggf. der darin gelösten Substanzen als Nähr- oder Schutzwirkstoffe an andere Körper, wie biologische Systeme, Saatgut, Pflanzen oder Mikroorganismen.

## Claims

1. An absorbent and swelling agent for water, aqueous solutions and body fluids, consisting of a physical mixture of at least two components, component A being at least one water-swellable, synthetic polymer or copolymer cross linked with a multifunctional compound, characterized in that component B is at least one polysaccharide from the group of galactomannans or polygalactomannans and galactomannan derivatives or mixtures thereof with other natural or synthetic polymerizates, which are flowable powders or fibrous materials at normal temperature and sparingly soluble or insoluble in water.

2. An absorbent and swelling agent for water, aqueous solutions and body fluids, which may be obtained from at least two components A and B, component A being at least one water-swellable, synthetic polymer or copolymer crosslinked with a multifunctional compound, component B being at least one polysaccharide from the group of galactomannans or polygalactomannans and galactomannan derivatives or mixtures thereof with other natural or synthetic polymerizates, which are flowable powders or fibrous materials at normal temperature and sparingly soluble or insoluble in water, by incorporating component B in a dried state or in a state from slightly moistened to swelled during the production of component A, after the polymerization conversion thereof has reached more than 60%, preferably more than 90%, and subsequent drying.

3. The absorbent and swelling agent according to claim 1 or 2, characterized in that said component A is a crosslinked polymer or copolymer based on acrylic acid, methacrylic acid, derivatives of these carboxylic acids, preferably a crosslinked homo- or copolymer of acrylic, methacrylic, acrylamidopropanesulfonic acid, the alkali or ammonium salts of these carboxylic acids, acryl- or methacrylamide and derivatives thereof, vinylpyrrolidone, as well as copolymers between each of these or with other, only partially water-soluble monomers such as vinyl acetate.

4. The absorbent and swelling agent according to claim 1 or 2, characterized in that said component B is a guar meal or locust bean seed meal alone or in mixture with other polysaccharides or polysaccharide derivatives, preferably mixtures of guar with starch, dextrin, dextran, cellulose and derivatives thereof.

5. The absorbent and swelling agent according to claim 1 or 2, characterized in that said component B is a guar meal or locust bean seed meal in mixture with natural or synthetic fibers, preferably mixtures with milled fibers based on cellulose, particularly mixtures with cotton or viscose fibers, or based on polymers, particularly mixtures with polyester, polyacrylonitrile or polyamide fibers.

6. The absorbent and swelling agent according to claim 2, characterized in that said component B is integrated in said synthetic polymer (component A) in such a way such that the water-extractable ratio of the final product is lower than that of a mixture, preferably lower than 30 wt.-%, more preferably lower than 20 wt.-%, relative to component B.

7. The absorbent and swelling agent according to one of claims 1 or 2, characterized in that the final product includes from 20 to 98 wt.-%, preferably from 50 to 90 wt.-% of component A, and from 2 to 80 wt.-%, preferably from 10 to 50 wt.-% of component B.

8. Use of the absorbent of claims 1 through 7 for absorbing and/or retaining water and/or aqueous solutions, particularly aqueous and serous body fluids such as urine or blood, in absorbent disposable articles for hygienic, surgical and medical purposes, such as diapers for babies, tampons and sanitary towels.

9. Use of the absorbent of claims 1 through 7 for medical purposes, such as oral administration as swelling agent, as an additive in contrast media, for regulating the intestinal peristalsis, for oral administration in order to retard the absorption or action of ingested drugs, or to reduce the blood cholesterol level.

10. Use of the absorbent of claims 1 through 7 in absorbing and/or retaining water or aqueous solutions, and in the subsequent controlled, retarded release of the absorbed liquid and optionally, the substances dissolved therein, as nutrients or protective agents to other bodies such as biological systems, seed, plants or micro-organisms.

## Revendications

1. Agents absorbants et agents gonflants pour l'eau, des solutions aqueuses et des liquides corporels, qui se composent d'un mélange physique d'au moins deux composants, parmi lesquels le composant A est formé d'au moins un copolymère ou un polymère réticulé avec un composé polyfonctionnel, synthétique, gonflable à l'eau, caractérisés en ce que le composant B est formé d'au moins un polysaccharide appartenant au groupe des galactomannanes et des polygalactomannanes, comme aussi les dérivés du galactomannane, ou leurs mélanges avec d'autres polymères naturels ou synthétiques, qui se présentent sous forme de matières fibreuses ou de poudres, fluides à la température normale et qui sont insolubles dans l'eau ou qui ne sont seulement solubles que de manière limitée dans l'eau.

2. Agents absorbants et agents gonflants pour l'eau, des solutions aqueuses et des liquides corporels, que l'on peut obtenir à partir d'au moins deux composants A et B, parmi lesquels le composant A est formé d'au moins un copolymère ou un polymère réticulé avec un composé multifonctionnel, synthétique, gonflable à l'eau et le composant B est formé d'au moins un polysaccharide appartenant au groupe des galactomannanes et des polygalactomannanes, ainsi que des dérivés du galactomannane ou de leurs mélanges avec d'autres polymères synthétiques ou naturels, qui se présentent sous forme de matières fibreuses ou de poudres fluides à la température normale et qui sont insolubles dans l'eau ou qui ne sont seulement solubles que de manière limitée dans l'eau, par l'incorporation du composant B à l'état séché ou à l'état légèrement humidifié à gonfler au cours de la préparation du composant A après que sa conversion par polymérisation a atteint plus de 60%, de préférence, plus de 90%, ainsi que par le séchage subséquent.

3. Agents absorbants et agents gonflants suivant la revendication 1 ou 2, caractérisés en ce que le composant A est un copolymère ou un polymère réticulé à base d'acide acrylique, d'acide méthacrylique, de dérivés de ces acides carboxyliques, de préférence, un copolymère ou un homopolymère réticulé de l'acide acrylique, de l'acide méthacrylique, de l'acide acrylamidopropanesulfonique, des sels de métaux alcalins ou d'ammonium de ces acides carboxyliques, de l'acrylamide ou du méthacrylamide et de leurs dérivés, de la vinylpyrrolidone, comme aussi de leurs copolymères entre eux ou avec d'autres monomères seulement partiellement solubles dans l'eau, comme, par exemple, l'acétate de vinyle.

4. Agents absorbants et agents gonflants suivant la revendication 1 ou 2, caractérisés en ce que le composant B est de la farine de gomme guar ou de la farine de graines de caroube, seules ou en mélange à d'autres polysaccharides ou dérivés de polysaccharides, de préférence, des mélanges de gomme guar avec de l'amidon, de la dextrine, du dextranne, de la cellulose et leurs dérivés.

5. Agents absorbants et agents gonflants suivant la revendication 1 ou 2, caractérisés en ce que le composant B est une farine de gomme guar ou une farine de graines de caroube en mélange à des fibres naturelles ou synthétiques, de préférence, des mélanges de fibres courtes à base de cellulose, plus particulièrement, des mélanges avec des fibres de coton ou de viscose, ou à base de polymère, plus spécialement des mélanges avec des fibres de polyester, de polyacrylonitrile ou de polyamide.

6. Agents absorbants et agents gonflants suivant la revendication 2, caractérisés en ce que le composant B est lié dans le polymère synthétique (composant A), en sorte que les fractions extractibles à l'eau du produit final soient inférieures que dans le cas d'un mélange, de préférence, inférieures à 30% en poids, de préférence, inférieures à 20% en poids, par rapport au composant B.

7. Agents absorbants et agents gonflants suivant la revendication 1 ou 2, caractérisés en ce que le produit final contient 20 à 98% en poids, de préférence, 50 à 90% en poids, de composant A et de 2 à 80% en poids, de préférence, 10 à 50% en poids, de composant B.

8. Utilisation de l'agent absorbant suivant l'une quelconque des revendications 1 à 7 pour la prise et/ou la rétention d'eau et/ou de solutions aqueuses, plus particulièrement, de liquides corporels aqueux et séreux, tels que l'urine ou le sang, dans des articles absorbants à jeter, pour des buts hygiéniques, chirurgicaux et médicaux, comme des couches pour bébés, des tampons et des serviettes hygiéniques.

9. Utilisation de l'agent absorbant suivant l'une quelconque des revendications 1 à 7 pour des buts médicaux, comme pour l'administration orale à titre d'agent gonflant, à titre d'additif pour agents de contraste, pour la régulation du péristaltisme intestinal, comme aussi pour l'administration orale dans le but de retarder la résorption et l'activité d'un médicament ingéré ou dans le but d'abaisser le taux de cholestérol sanguin.

10. Utilisation de l'agent absorbant suivant l'une quelconque des revendications 1 à 7 pour la prise et/ou la rétention d'eau ou de solutions aqueuses en vue de la délivrance subséquente, réglée, retardée du liquide absorbé, comme éventuellement aussi des substances qui s'y trouvent dissoutes, à titre de principes nutritifs ou protecteurs à d'autres corps, comme des systèmes biologiques, des semences, des plantes ou des micro-organismes.
